# EUROPEAN PATENT APPLICATION

(11) **EP 4 681 638 A1**
(43) Date of publication of application: **21.01.2026**
(21) Application number: 25183869.4
(22) Date of filing: 18.06.2025
(51) Int. Cl.: A61B 5/1455

(54) **PIGMENTATION INSENSITIVE PULSE OXIMETER**

(30) Priority: 17.07.2024 US 202418775095
(71) Applicant: Zynex Monitoring Solutions, Inc., Englewood, CO 80112 (US)
(72) Inventor: Pologe, Jonas Alexander, Englewood, 80112 (US); Watkins, Connor James, Englewood, 80112 (US)
(74) Representative: Forresters IP LLP

(57) **Abstract**

A photoplethysmographic sensor has a first light source, a second light source, an optical filter positioned within the path of the light emitted by the first light source and adapted to pass light within the spectral bandwidth of the first light source, an output aperture positioned to allow light passing through the optical filter and light emitted by the second light source to pass through, and a photodetector configured to detect light from the first and second light sources and to convert the detected light signal into an electronic signal. The first light source, the optical filter, and the output aperture configured to prevent light output by the first light source from passing through the output aperture without first passing through the optical filter.

## Description

### FIELD OF THE INVENTION

This invention is in the field of photoplethysmography.

### BACKGROUND OF THE INVENTION

In the science of photoplethysmography, light is used to illuminate or trans-illuminate living tissue for the purpose of providing noninvasive measurements of blood analytes including but not limited to the levels of arterial oxyhemoglobin, carboxyhemoglobin, methemoglobin, reduced hemoglobin, and/or total hemoglobin. Additionally, photoplethysmography can be designed to measure various hemodynamic parameters, and/or tissue properties including, but not limited to, heart rate, respiratory rate, and perfusion.

In this monitoring modality multiple different spectral bands of light are directed into or incident on living tissue (the "tissue-under-test") and a portion of the light that is not absorbed by the tissue or scattered in some other direction is detected by a photodetector a short distance from the point at which the light entered the tissue. When light, at wavelengths that can be absorbed by hemoglobin or other components of arterial blood, passes through living tissue the light is modulated by the pulsatile arterial blood flow. The pulsatile (or photoplethysmographic) light signals exiting the tissue and picked up by the photodetector are converted into electronic signals (or photoplethysmographic signals or photoplethysmographic data) and are then used to calculate the desired blood analyte levels and/or hemodynamic parameters. A device which generates light to be emitted into the tissue and detects and processes the photoplethysmographic signals (or data) emitted by the tissue, to measure the levels of various blood analytes and/or various hemodynamic parameters, is a photoplethysmographic device. A photoplethysmographic device typically includes a photoplethysmographic monitor combined with a photoplethysmographic sensor. The first widely used commercial photoplethysmographic device was a pulse oximeter, a photoplethysmographic device designed to noninvasively measure, at least, arterial blood oxygen saturation. This device is now used in almost all areas of medicine.

The (photoplethysmographic) monitor includes electronic circuitry for controlling light emitters that emit light which is then incident on the tissue. The monitor also performs the functions of processing the photoplethysmographic signals emitted from the tissue and picked up by the photodetector, converting these photoplethysmographic data into the various blood analytes and/or hemodynamic parameter measurements, and displaying these measurements on some form of user display. Also included in any photoplethysmographic device is a sensor which is affixed to, or held in place against, the tissue to deliver light from the emitters to the tissue. The sensor further includes the photodetector for receiving the photoplethysmographic signals from the tissue.

Typically, the sensor is connected to the monitor by a patient cable that has a connector to allow it to be removably connected to the monitor. Further, depending on the design of the patient cable, the sensor can be permanently connected to the distal end of patient cable, or the patient cable may have a connector on its distal end to connect and disconnect to the sensors.

In conventional pulse oximeter sensors, two (or more) light emitting diodes (LEDs) are typically employed which generate the light used to probe the tissue. One LED commonly has a center wavelength around 660 nanometers (nm), in the red portion of the visible spectrum, and a second LED has a center wavelength typically around 900nm or 940nm, in the near infrared region. These LEDs are typically located in the sensor which, when placed on the tissue, positions the LEDs within a few millimeters of the tissue they illuminate. This generates a strong optical signal for probing the tissue. The photodetector, for detecting a portion of the light exiting the tissue, is also typically positioned directly in the sensor to be held in close proximity to the tissue when in use.

LED-based light sources in photoplethysmography, when commercially introduced in the early 1980s, revolutionized pulse oximetry by providing intense light sources centered at the desired wavelengths. But these light sources created a new problem clinically. LED-based pulse oximeters read falsely higher arterial oxygen saturation levels on patients with high levels of melanin in the tissue-under-test. These faulty readings have caused an increase in the frequency of missed diagnoses of hypoxemia in darkly pigmented patients compared with patients that are more lightly pigmented.

This problem occurs due to two factors. First, the red LEDs used in pulse oximeters are somewhat broadband, having a full-power spectral bandwidth approaching 100nm. Second, melanin has a steeply-sloped optical absorption across the spectral bandwidth of the red LEDs used in pulse oximeters. Melanin absorbs more strongly at the shorter wavelengths of the red LED light than at the longer wavelengths. This effectively "shifts" the spectrum of light received by the photodetector from the red LED toward the longer wavelengths where reduced hemoglobin has a lower extinction causing the pulse oximeter to read a higher oxygen saturation than is actually present in the patient.

### SUMMARY OF THE INVENTION

The present invention overcomes the problems and disadvantages associated with current strategies and designs and provides new systems and methods of manufacturing photoplethysmographic sensors.

A preferred embodiment of the invention is directed to a photoplethysmographic sensor. The sensor has a first light source adapted to emit light with a spectral bandwidth greater than 30nm at 5% of peak intensity and a peak intensity in the wavelength range of 620nm to 770nm, a second light source adapted to emit light with a peak intensity in the wavelength range of 800nm to 960nm, an optical filter positioned within the path of the light emitted by the first light source and adapted to pass light within the spectral bandwidth of the first light source, the optical filter comprising a bandpass filter with a passband less than 30nm at 5% of peak of the passband, the optical filter adapted to attenuate light incident on the optical filter at wavelengths outside the passband by at least 85%, an output aperture positioned to allow light passing through the optical filter and light emitted by the second light source to pass through, the first light source, the optical filter, and the output aperture configured to prevent light output by the first light source from passing through the output aperture without first passing through the optical filter, a photodetector configured to detect light from the first and second light sources and to convert the detected light signal into an electronic signal.

Preferably, a housing contains the output aperture and the photodetector. In a preferred embodiment, the first light source is a light-emitting diode and/or the second light source is a light-emitting diode. Preferably, the optical filter comprises two or more filters to provide a bandpass less than 20nm. The sensor preferably further comprises opaque walls positioned between the first emitter and the optical filter to constrain the output aperture from passing light emitted by the first emitter that has not first passed through the optical filter. Preferably, the sensor further comprises a diffuser adapted to diffuse light from the first light source after said light passes through the optical filter and light from the second light source before either light is passed to the output aperture.

Another embodiment of the invention is directed to a method of assembling a photoplethysmographic sensor. The method includes positioning a first light source adapted to emit light with a spectral bandwidth greater than 30nm at 5% of peak intensity and with a peak intensity in the wavelength range of 620nm to 770nm in the sensor, positioning a second light source adapted to emit light with a peak intensity in the wavelength range of 800nm to 960nm in the sensor, positioning an output aperture in the sensor, wherein the output aperture is positioned to pass light emitted from the first light source and the second light source, positioning an optical filter between the first light source and the output aperture to prevent light output by the first light source from passing through the output aperture without passing through the optical filter, wherein the optical filter is configured to pass light within the spectral bandwidth of the first light source and with a passband less than 30nm, the optical filter further configured to attenuate light incident on the optical filter at wavelengths outside the passband by at least 85%, and positioning a photodetector configured to detect light from the first and second light sources and to convert the detected light signal into an electronic signal.

Preferably at least the output aperture and the photodetector are positioned within a housing. In a preferred embodiment, the first and/or second light source comprises a light-emitting diode. Preferably, the optical filter comprises two or more filters to provide a bandpass less than 20nm. The method preferably further comprises positioning opaque walls between the first emitter and the optical filter to constrain the output aperture from passing any light emitted by the first emitter that has not first passed through the optical filter. Preferably, the method further comprises positioning a diffuser in the sensor, wherein the diffuser is adapted to diffuse light from the first light source after said light passes through the optical filter and light from the second light source before the light from either light source is passed to the output aperture.

Other embodiments and advantages of the invention are set forth in part in the description, which follows, and in part, may be obvious from this description, or may be learned from the practice of the invention.

### BRIEF DESCRIPTION OF THE DRAWINGS

The foregoing features of embodiments will be more readily understood by reference to the following detailed description, taken with reference to the accompanying drawings, which show:
*FIG. 1**:* Basic photoplethysmographic sensor.
*FIG. 2*: Emitter section of photoplethysmographic sensor with diffuser and optically isolated LED.
*FIG. 3*: Photoplethysmographic sensor with separable emitter section.

### DETAILED DESCRIPTION OF THE INVENTION

As embodied and broadly described herein, the disclosures herein provide detailed embodiments of the invention. However, the disclosed embodiments are merely exemplary of the invention that may be embodied in various and alternative forms. Therefore, there is no intent that specific structural and functional details should be limiting, but rather the intention is that they provide a basis for the claims and as a representative basis for teaching one skilled in the art to variously employ the present invention.

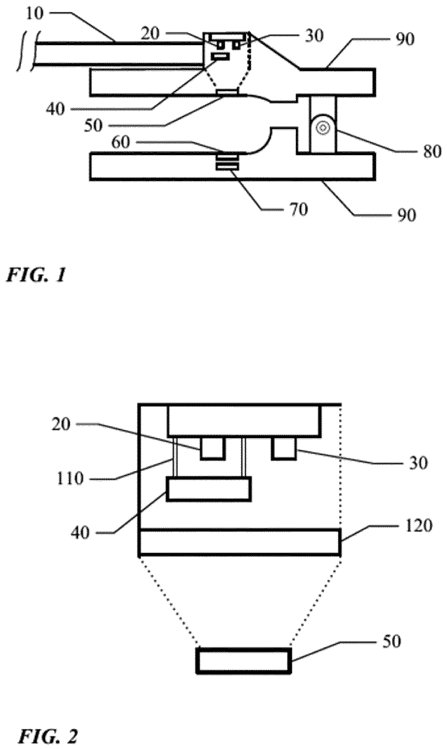

Photoplethysmographic sensors come in many forms including, but not limited to that shown schematically in Fig. 1. These sensors provide a way to apply light signals, generated by a red LED 20 and a near infrared (NIR) LED 30, to the tissue-under-test and to receive a portion of these light signals exiting the tissue at photodetector **70.** Light emitted by the sensor into the tissue is highly scattered by the red blood cells in the tissue and absorbed by the hemoglobin in the red blood cells. Only a small portion of the light, emitted by LEDs **20** and **30,** entering the tissue exits the tissue at the location where photodetector **70** is held against the tissue by the sensor. The light that strikes photodetector **70** is converted into an electronic signal and that electronic signal is communicated (or transmitted) back to the photoplethysmographic device for processing into the desired measurements. Light that passes through living tissue with pulsatile blood flow will be modulated by the pulsatile increase and decrease in optical density due to the pulsatile changes in the blood volume of the tissue between LEDs **20** and **30** and photodetector **70.** Consequently, the light exiting the tissue will be pulsatile and these pulsatile light signals, received by photodetector **70,** are referred to as photoplethysmographic signals.

A receiving aperture **60** constrains the light out of the tissue that can be incident on the photodetector **70.** In some embodiments, positioning receiving aperture **60** over photodetector **70** is not required in the sensor because a photodetector held in close proximity to the tissue-under-test will effectively create an aperture for the light by itself without the need for a separate aperture element. Further, in some embodiments, receiving aperture **60** and photodetector **70** in the sensor may be replaced by a light guide, such as a fiber optic bundle, that communicates the received light signal to a photodetector positioned in a different location, for example inside the photoplethysmographic monitor. This fiber optic configuration is to be considered the same as having photodetector **70** located in the sensor.

Using the full spectrum of light emitted by the first light source, red LED **20,** causes conventional pulse oximeters to read falsely elevated oxygen saturation levels on hypoxemic (low arterial oxygen saturation) patients that have highly melanized tissue. The red LEDs utilized in pulse oximetry typically have a peak wavelength (the wavelength where the light out of an LED is at its peak intensity) in the range of 650nm (nanometers) to 675nm. Note, this first light source may have a peak intensity anywhere from 620nm to 770nm. These red LEDs also typically have a full power spectral bandwidth that can exceed 70nm. Even at 5% of the peak LED intensity these red LEDs have a spectral bandwidth that exceeds 30nm.

Because melanin absorbs the shorter wavelength content of the output light of the red LEDs used in pulse oximetry more strongly than the longer wavelengths of the red LEDs, the center wavelength of the red LED light passed through highly melanized tissue-under-test is shifted towards the longer wavelengths by several nanometers. The greater the shift in the spectral content of the red LED by melanin in the tissue, the greater the error in the measured oxygen saturation by a pulse oximeter. For example, a patient with a true oxygen saturation of 70% may read several percent higher just due to the presence of melanin in the tissue-under-test.

To overcome this flaw in conventional photoplethysmographic design, the sensor of FIG. 1 incorporates an optical filter **40** to constrain the output light of the first light source, the red LED **20,** to a narrower spectral bandwidth. Optical filter **40** preferably has a spectral bandpass less than 30nm at the 5% level of the peak of the passband. Ideally this bandpass is preferably as narrow as possible because the narrower the spectral bandwidth of the red LED light passing through optical filter **40** the smaller the spectral shift that the melanin can induce on this light and therefore the smaller the error in oxygen saturation measurement. However, the narrower the passband, the lower total light intensity that is passed through to the tissue-under-test. This decreases the signal-to-noise ratio of the measurement, and thus there is a tradeoff in passband selection of optical filter **40.** Passbands, at the 5% of the peak level, in the 5nm, 10nm, 15nm, 20nm, 25nm, and 30nm range can be used. The shorter the passband, the brighter the red LED should be to minimize the degradation in the signal-to-noise.

Optical filter **40** preferably comprises an interference filter made up of a thin glass substrate (or other substrate material transparent to the wavelengths in the desired passband) coated with multiple thin layers of dielectric material. The specific design of the multi-layer filter employed allows certain wavelengths of light to pass through with minimal attenuation while blocking or reflecting wavelengths outside the desired passband.

The sensor of FIG. 1 is preferably configured so that only light output by red LED **20** that passes through optical filter **40** can pass to output aperture **50.** A second light source **30** outputs light in the near infrared (NIR) region with a peak intensity in the 800nm to 960nm range. As shown in FIG. 1, NIR LED **30** is positioned within the sensor to allow its output light to pass directly from NIR LED **30** to output aperture **50** without passing through optical filter **40.**

Note that physically separating light source(s) **20** and/or **30** from optical filter **40** or output aperture **50,** for example through the use of light guides such as fiber optics to conduct light from light source(s) **20** and/or **30** to optical filter **40** and/or output aperture **50,** is considered to be the same as having light sources **20** and/or **30** in the sensor.

As with most reusable photoplethysmographic sensors, the sensor shown in FIG. 1 also includes a housing **90,** often made of plastic, that holds the rest of the elements of the sensor including LED emitters **20** and **30,** the optical filter **40,** output aperture **50,** receiving aperture **60,** and the photodetector **70.** The housing shown in FIG.1 has a hinge **80,** typically spring-loaded, which allows the upper and lower halves of the sensor housing **90** to open to accept the tissue-under-test, for example a finger, to be inserted and then to close gently on the tissue and hold the sensor in place. The sensor shown here further includes a cable **10** which contains the wires that connect to LEDs **20** and **30** and photodetector **70.** The proximal end (not shown) of cable **10** typically connects the sensor to the photoplethysmographic monitor, most often through a connector that removably connects to the monitor.

FIG. 2 is a close-up view of the emitter portion of the sensor shown in FIG. 1. The emitter portion shown in FIG. 2 retains all the elements of the emitter portion shown in FIG. 1 and adds optional walls **110** and a diffuser **120.** Walls **110** are optically opaque elements, positioned within the emitter section of the sensor, to block any light emitted by red LED **20** that does not pass through optical filter **40** from exiting through output aperture **50.** The walls can create an enclosure between red LED **20** and optical filter **40** and can be made of metal, carbon filled epoxy, plastic, or any number of other materials as long as walls **110** are sufficiently opaque to light at the wavelengths emitted by red LED **20.**

Diffuser **120** is adapted to diffuse the light emitted by LEDs **20** and **30.** This element helps to ensure that the light from each LED **20** and **30** exiting output aperture **50** is reasonably homogeneously distributed across output aperture **50.** The addition of walls **110** can increase the physical separation between LEDs **20** and **30** making diffuser **120** more necessary to ensure accurate readings.

Diffuser **120** is less crucial if LEDs **20** and **30** can be placed very close together in the emitter section of the sensor and when the tissue-under-test is thick, such as with a finger. Diffuser **120** is more important if the separation between LEDs **20** and **30** is greater and the tissue-under-test is thinner, such as with an earlobe.

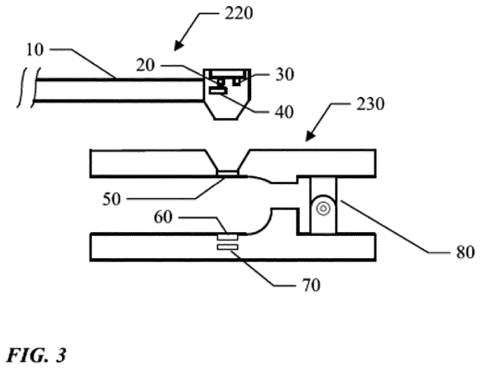

Fig. 3 is a schematic representation of a sensor with a separable sensor housing **230** that can separate from a patient cable **220.** In the embodiment shown, the emitter portion of the sensor is permanently attached to cable **10.** When patient cable **220** is snapped into sensor housing **230** the sensor operates as described herein.

The sensor designs shown in FIGs. 1 and 3 are just two examples of the many sensor configurations currently employed in photoplethysmography. Other examples include disposable sensors where the sensor housing is primarily made up of cloth and tape, reusable sensors made with soft rubber housings, and sensors with silicon housings that are taped in place on the tissue.

Other embodiments and uses of the invention will be apparent to those skilled in the art from consideration of the specification and practice of the invention disclosed herein. It is intended that the specification and examples be considered exemplary only with the true scope of the invention indicated by the following claims. Furthermore, the term "comprising of" includes the terms "consisting of" and "consisting essentially of."

### REPRESENTATIVE FEATURES

Representative features are set out in the following clauses, which stand alone or may be combined, in any combination, with one or more features disclosed in the text and/or drawings of the specification.
1. A photoplethysmographic sensor comprising:
   a first light source adapted to emit light with a spectral bandwidth greater than 30nm at 5% of peak intensity;
   the first light source adapted to emit light with a peak intensity in the wavelength range of 620nm to 770nm;
   a second light source adapted to emit light with a peak intensity in the wavelength range of 800nm to 960nm;
   an optical filter positioned within the path of the light emitted by the first light source and adapted to pass light within the spectral bandwidth of the first light source;
   the optical filter comprising a bandpass filter with a passband less than 30nm at 5% of peak of the passband;
   the optical filter adapted to attenuate light incident on the optical filter at wavelengths outside the passband by at least 85%;
   an output aperture positioned to allow light passing through the optical filter and light emitted by the second light source to pass through;
   the first light source, the optical filter, and the output aperture configured to prevent light output by the first light source from passing through the output aperture without first passing through the optical filter; and
   a photodetector configured to detect light from the first and second light sources and to convert the detected light signal into an electronic signal.
2. The sensor of clause **1,** wherein a housing contains the output aperture and the photodetector.
3. The sensor of clause 1 or 2, wherein the first light source is a light-emitting diode.
4. The sensor of any one of the preceding clauses, wherein the second light source is a light-emitting diode.
5. The sensor of any one of the preceding clauses, wherein the optical filter comprises two or more filters to provide a bandpass less than 20nm.
6. The sensor of any one of the preceding clauses, further comprising opaque walls positioned between the first emitter and the optical filter to constrain the output aperture from passing light emitted by the first emitter that has not first passed through the optical filter.
7. The sensor of any one of the preceding clauses, further comprising a diffuser adapted to diffuse light from the first light source after said light passes through the optical filter and light from the second light source before either light is passed to the output aperture.
8. A method of assembling a photoplethysmographic sensor, comprising the steps of:
   positioning a first light source adapted to emit light with a spectral bandwidth greater than 30nm at 5% of peak intensity and with a peak intensity in the wavelength range of 620nm to 770nm in the sensor;
   positioning a second light source adapted to emit light with a peak intensity in the wavelength range of 800nm to 960nm in the sensor;
   positioning an output aperture in the sensor, wherein the output aperture is positioned to pass light emitted from the first light source and the second light source;
   positioning an optical filter between the first light source and the output aperture to prevent light output by the first light source from passing through the output aperture without passing through the optical filter;
   wherein the optical filter is configured to pass light within the spectral bandwidth of the first light source and with a passband less than 30nm, the optical filter further configured to attenuate light incident on the optical filter at wavelengths outside the passband by at least 85%; and
   positioning a photodetector configured to detect light from the first and second light sources and to convert the detected light signal into an electronic signal.
9. The method of clause 8, wherein at least the output aperture and the photodetector are positioned within a housing.
10. The method of clause 8 or 9, wherein the first light source comprises a light-emitting diode.
11. The method of any one of clauses 8 to 10, wherein the second light source comprises a light-emitting diode.
12. The method of any one of clauses 8 to 11, wherein the optical filter comprises two or more filters to provide a bandpass less than 20nm.
13. The method of any one of clauses 8 to 12, further comprising positioning opaque walls between the first emitter and the optical filter to constrain the output aperture from passing any light emitted by the first emitter that has not first passed through the optical filter.
14. The method of any one of clauses 8 to 13, further comprising positioning a diffuser in the sensor, wherein the diffuser is adapted to diffuse light from the first light source after said light passes through the optical filter and light from the second light source before the light from either light source is passed to the output aperture.

## Claims

1. A photoplethysmographic sensor comprising:
a first light source adapted to emit light with a spectral bandwidth greater than 30nm at 5% of peak intensity;
the first light source adapted to emit light with a peak intensity in the wavelength range of 620nm to 770nm;
a second light source adapted to emit light with a peak intensity in the wavelength range of 800nm to 960nm;
an optical filter positioned within the path of the light emitted by the first light source and adapted to pass light within the spectral bandwidth of the first light source;
the optical filter comprising a bandpass filter with a passband less than 30nm at 5% of peak of the passband;
the optical filter adapted to attenuate light incident on the optical filter at wavelengths outside the passband by at least 85%;
an output aperture positioned to allow light passing through the optical filter and light emitted by the second light source to pass through;
the first light source, the optical filter, and the output aperture configured to prevent light output by the first light source from passing through the output aperture without first passing through the optical filter; and
a photodetector configured to detect light from the first and second light sources and to convert the detected light signal into an electronic signal.

2. The sensor of claim 1, wherein a housing contains the output aperture and the photodetector.

3. The sensor of claim 1 or 2, wherein the first light source is a light-emitting diode.

4. The sensor of any one of the preceding claims, wherein the second light source is a light-emitting diode.

5. The sensor of any one of the preceding claims, wherein the optical filter comprises two or more filters to provide a bandpass less than 20nm.

6. The sensor of any one of the preceding claims, further comprising opaque walls positioned between the first emitter and the optical filter to constrain the output aperture from passing light emitted by the first emitter that has not first passed through the optical filter.

7. The sensor of any one of the preceding claims, further comprising a diffuser adapted to diffuse light from the first light source after said light passes through the optical filter and light from the second light source before either light is passed to the output aperture.

8. A method of assembling a photoplethysmographic sensor, comprising the steps of:
positioning a first light source adapted to emit light with a spectral bandwidth greater than 30nm at 5% of peak intensity and with a peak intensity in the wavelength range of 620nm to 770nm in the sensor;
positioning a second light source adapted to emit light with a peak intensity in the wavelength range of 800nm to 960nm in the sensor;
positioning an output aperture in the sensor, wherein the output aperture is positioned to pass light emitted from the first light source and the second light source;
positioning an optical filter between the first light source and the output aperture to prevent light output by the first light source from passing through the output aperture without passing through the optical filter;
wherein the optical filter is configured to pass light within the spectral bandwidth of the first light source and with a passband less than 30nm, the optical filter further configured to attenuate light incident on the optical filter at wavelengths outside the passband by at least 85%; and
positioning a photodetector configured to detect light from the first and second light sources and to convert the detected light signal into an electronic signal.

9. The method of claim 8, wherein at least the output aperture and the photodetector are positioned within a housing.

10. The method of claim 8 or 9, wherein the first light source comprises a light-emitting diode.

11. The method of any one of claims 8 to 10, wherein the second light source comprises a light-emitting diode.

12. The method of any one of claims 8 to 11, wherein the optical filter comprises two or more filters to provide a bandpass less than 20nm.

13. The method of any one of claims 8 to 12, further comprising positioning opaque walls between the first emitter and the optical filter to constrain the output aperture from passing any light emitted by the first emitter that has not first passed through the optical filter.

14. The method of any one of claims 8 to 13, further comprising positioning a diffuser in the sensor, wherein the diffuser is adapted to diffuse light from the first light source after said light passes through the optical filter and light from the second light source before the light from either light source is passed to the output aperture.
